# EUROPEAN PATENT SPECIFICATION

(11) **EP 3 448 529 B1**
(45) Date of publication and mention of the grant of the patent: **18.10.2023**
(21) Application number: 17790044.6
(22) Date of filing: 15.03.2017
(51) Int. Cl.: A61Q 19/00, A61K 8/368, A61K 31/60, A61L 26/00, A61P 17/10, A61K 9/70, A61K 31/327, A61K 33/04

(54) **MEDICATED HYDROCOLLOID DRESSING FOR ACNE TREATMENT**
MEDIZINISCHER HYDROKOLLOIDVERBAND ZUR AKNEBEHANDLUNG
PANSEMENT HYDROCOLLOÏDE MÉDICAMENTEUX POUR TRAITEMENT DE L'ACNÉ

(30) Priority: 26.04.2016 US 201662327879 P; 03.06.2016 US 201662345096 P
(43) Date of publication of application: 06.03.2019
(73) Proprietor: Peace Out Inc., San Francisco CA 94103 (US)
(72) Inventor: FREZZA, Enrico, Mill Valley, CA 94941 (US)
(74) Representative: CH Kilger Anwaltspartnerschaft mbB
(86) International application number: PCT/US2017/022499
(87) International publication number: WO 2017/189112

(56) References cited:
- WO-A1-01/13968
- WO-A1-01/13968
- US-A1- 2005 080 465
- US-A1- 2005 244 525
- US-A1- 2006 253 078
- US-A1- 2014 363 488
- US-B1- 6 495 158
- ASHLEY DECKER: "Over-the-counter Acne Treatments", J CLIN AESTHET DERMATOL., vol. 5, no. 5, 1 May 2012 (2012-05-01), pages 32-40, XP055644876,

## Description

### FIELD OF THE INVENTION

The present technology relates generally to devices for acne treatment, as well as methods for preparing the devices and methods of treatment using the devices. Suitably, the devices comprise a hydrocolloid dressing and an active agent, wherein the amount of the active agent does not reduce the absorption properties of the hydrocolloid dressing. In exemplary embodiments, the active agent is salicylic acid, benzoyl peroxide or sulfur.

The references to methods of treatment in this description are to be interpreted as references to the compounds, pharmaceutical compositions and medicaments of the present invention for use in a method for treatment of the human (or animal) body by therapy (or for diagnosis)

### BACKGROUND OF THE INVENTION

The main problem in the treatment of acne is that the over-the-counter, active ingredients, recognized and permitted by the FDA for the treatment of acne are hardly ever effective. One reason for this is because a cream or topical solution applied to the skin is still exposed to the external environment. This allows for bacteria and contaminants to be absorbed onto the skin and the acne blemish, which causes inflammation and slows down the healing process of acne tremendously. In addition, these active ingredients alone tend to over dry the skin which causes acne scars.

While hydrocolloid dressings exist for the treatment of acne, they do not contain medication to further aid the treatment process, relying solely on the absorption properties of the dressing.

Buseman et al. (US patent 6,495,158 B1) published an adhesive patch with a flexible backing and an embedded therapeutic formulation for treatment of acne. However, for the device of the present invention the formulation of the hydrocolloid dressing and the active agent are optimized to not reduce absorption capability for oil, pus, dirt, bacteria and other materials from the skin surface and an acne blemish. Thus the active agents of the present invention achieve improved efficacy.

Lipman et al. (patent application WO 01/13968 A1) disclosed pressure sensitive hydrocolloid adhesives comprising cyclodextrin, which are useful in medical products including antibacterial adhesives or adhesives that bleach the skin, prevent and diminish acne skin blemishes. However, the adhesives of Liman et al. also do not describe the optimization to not reduce absorption capability and thus achieve improved efficacy.

Decker et al. (J Clin Aesthet Dermatol 2012;5(5):32-40) published an review on available acne treatments in particular the active agents which are effective in acne treatments.

### BRIEF SUMMARY OF THE INVENTION

In view of the foregoing, provided herein are devices and methods for treating acne which utilize the absorption properties of a hydrocolloid dressing in combination with an active agent.

Embodiments hereof are directed to devices for treatment of acne, comprising a removable layer on an inner surface of the device; and a flexible sheet or wafer consisting of a) a hydrocolloid dressing comprising a polyurethane foam or film, and an active agent selected from the group consisting of salicylic acid, benzoyl peroxide and sulfur associated with the hydrocolloid dressing, wherein the active agent is present at an amount so as to not reduce the absorption capability of the hydrocolloid dressing, wherein benzoyl peroxide is present at an amount of 2.5% to 10%, or the sulfur is present at an amount of 3% to 10%.

In exemplary embodiments, the active agent is salicylic acid present at an amount of 0.5% to 2%, or 0.5% to 1.2%. In additional embodiments, the active agent is benzoyl peroxide present at an amount of 2.5% to 10%, or in further embodiments, the active agent is sulfur present at an amount of 3% to 10%.

In additional embodiments, an inner surface of the device further comprises an adhesive for adhering to the skin surface.

Also provided herein are devices for treatment of acne, consisting essentially of a hydrocolloid dressing, and salicylic acid present at an amount of 0.5% to 1.5%, wherein the hydrocolloid dressing comprises a polyurethane foam or film.

In further embodiments, provided herein are systems for treatment of acne, comprising a plurality of devices, each device comprising, a removable layer on an inner surface of the device; and a flexible sheet or wafer consisting of i) a hydrocolloid dressing comprising a polyurethane foam or film, an active agent selected from the group consisting of salicylic acid, benzoyl peroxide and sulfur associated with the hydrocolloid dressing, wherein the active agent is present at an amount so as to not reduce the absorption capability of the hydrocolloid dressing, and a removable layer covering an inner surface of device, wherein each device is in the form of an application pad having a size of 0.5 cm to 4 cm.

In embodiments, plurality of devices are separate, and each comprises the removable layer covering the inner surface of the device, or in additional embodiments, at least two of the devices are attached to the same removable layer. Suitably, the plurality of devices have square, circle or oval shapes.

Also provided herein are methods of treating acne according to the disclosure, comprising providing a device, the device comprising, a hydrocolloid dressing, and an active agent selected from the group consisting of salicylic acid, benzoyl peroxide and sulfur associated with the hydrocolloid dressing, wherein the active agent is present at an amount so as to not reduce the absorption capability of the hydrocolloid dressing. The methods further comprise applying the device to a skin surface, the device at least partially covering an acne blemish, and maintaining the device on the skin surface for a period of at least 1 hour, thereby treating the acne.

In suitable embodiments, the acne blemish is a blackhead or a whitehead.

Suitably, the devices is maintained ice on the skin surface for a period of at least 6 hours, and in exemplary embodiments, the method of treatment is repeated daily for a period of at least 3 days.

In additional embodiments, the device further comprises a removable layer on an inner surface of the device, and the method further comprises removing the removable layer prior to applying the device to the skin surface.

Also provided herein are methods of preparing a device for treatment of acne, comprising, providing a solution of an active agent selected from the group consisting of salicylic acid, benzoyl peroxide and sulfur, mixing the solution of the active agent with a paste comprising a hydrocolloid, the mixing conducted at 20-30°C, to form a mixture, and applying the mixture to a removable layer to generate the device, wherein the active agent is present at an amount so as to not reduce the absorption capability of the hydrocolloid dressing.

In suitable embodiments, the amount of salicylic acid is 0.5% to 1.5%.

The methods can also further comprise cutting the device into a plurality of separate devices.

Also provided herein are devices for treatment of acne prepared by the processes described herein.

### BRIEF DESCRIPTION OF DRAWINGS

The foregoing and other features and aspects of the present technology can be better understood from the following description of embodiments and as illustrated in the accompanying drawings. The accompanying drawings, which are incorporated herein and form a part of the specification, further serve to illustrate the principles of the present technology. The components in the drawings are not necessarily to scale.
FIG. 1 shows a sectional view of a device for treatment of acne as described in embodiments herein.
FIGS. 2A-2D show sectional views of devices for treatment of acne as described in embodiments herein.
FIGS. 3A-3B show sectional views of devices for treatment of acne as described in embodiments herein.
FIGS. 4A-4B show systems as described in embodiments herein.
FIG. 5 illustrates a method of treating acne as described in embodiments herein as according to the disclosure.
FIG. 6 illustrates a further system as described in embodiments herein.

### DETAILED DESCRIPTION OF THE INVENTION

It should be appreciated that the particular implementations shown and described herein are examples and are not intended to otherwise limit the scope of the application in any way.

Any conflict between any reference cited herein and the specific teachings of this specification shall be resolved in favor of the latter. Likewise, any conflict between an art-understood definition of a word or phrase and a definition of the word or phrase as specifically taught in this specification shall be resolved in favor of the latter.

As used in this specification, the singular forms "a," "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

Technical and scientific terms used herein have the meaning commonly understood by one of skill in the art to which the present application pertains, unless otherwise defined. Reference is made herein to various methodologies and materials known to those of skill in the art.

In embodiments, provided herein are hydrocolloid dressings with FDA recognized and permitted acne active ingredients which allows for use an as acne treatment.

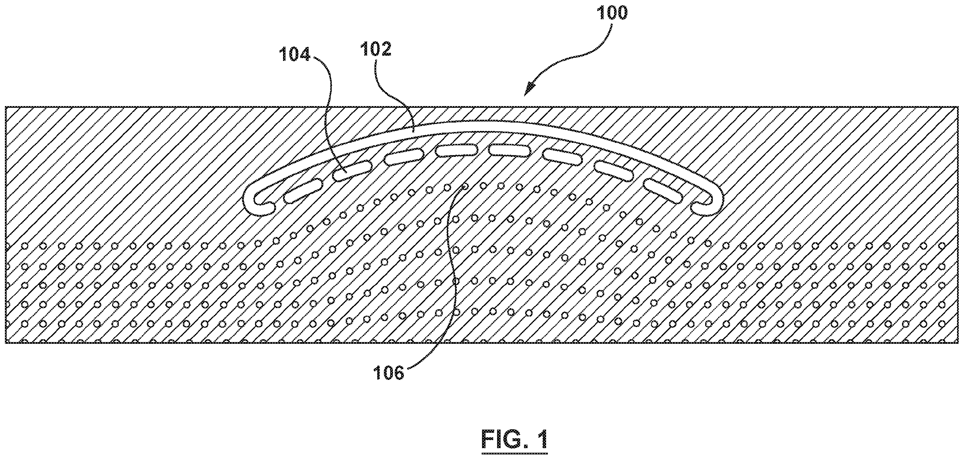

In exemplary embodiments, as shown in FIG. 1, provided herein are devices 100 for treatment of acne. In embodiments, device 100 suitably comprises a hydrocolloid dressing 102 and an active agent 104 associated with the hydrocolloid dressing. In exemplary embodiments, the active agent is an FDA agent approved for treatment of acne, suitably selected from the group consisting of salicylic acid, benzoyl peroxide and sulfur.

The structure of device 100 in FIG. 1 is provided for illustrative purposes only and is not to be construed to provide the only structure or orientation for the device described herein.

As described herein, the active agent is present at an amount so as to not reduce the absorption capability of the hydrocolloid dressing. It has been surprisingly discovered that by properly formulating the hydrocolloid dressing and active agent, the ability of the hydrocolloid dressing to absorb oil, pus, dirt, bacteria and other materials from the skin surface and an acne blemish is not reduced or impacted, i.e., the "absorption capability" of the hydrocolloid dressing is not reduced.

As used herein, a "hydrocolloid dressing" refers to a flexible sheet or wafer which contains material that forms a gel in the presence of water. Hydrocolloid dressings are suitably biodegradable, non-breathable, and can adhere to the skin without the use of adhesives or separate taping.

The active surface of a hydrocolloid dressing is coated with a cross-linked adhesive mass containing a dispersion of gelatin, pectin and carboxy-methylcellulose (e.g., sodium carboxymethylcellulose (NaCMC) together with other polymers and adhesives. Suitably the hydrocolloid is combined with elastomers and adhesives and applied to a carrier for example a polyurethane foam or film, to form an absorbent, self adhesive, waterproof wafer. In contact with a fluid (e.g., a wound exudate, pus, oil, blood, etc.), the polysaccharides and other polymers absorb water and swell, forming a gel which is held within the structure of the adhesive matrix. The gel which is formed as a result of the absorption of fluid is not mobile and free running but held within the structure of the adhesive matrix. Suitably, a hydrocolloid dressing is waterproof.

In suitable embodiments, hydrocolloid dressing is a hi-tack dressing, which can comprise a hydrophobic pressure sensitive adhesive (e.g., butyl rubber or a styrene-isoprene-styrene (SIS) block copolymer) compounded with hydrophilic particulates. As described herein, suitably the hydrocolloid dressing comprises a polyurethane carrier covering the noncontacting side of the dressing. In other embodiments, hydrocolloid dressing can comprise cis-1,4-polyisoprene (Natural rubber) polybutadiene (butyl rubber) thermoplastic elastomer block copolymers, copolymers of alkyl acrylates. The hydrocolloid dressing suitably demonstrate a removable, high initial tack (i.e., high adhesion to a skin surface), which has a general-purpose removable adhesive with good internal strength, and stable adhesion. The dressings remove cleanly from most surfaces, including skin.

The devices described herein also aid in correcting any abnormal shedding of acne which helps to unclog pores and absorb the exudate of acne.

The percentage of active agent is measured as a weight/weight measurement based on the total weight of the hydrocolloid dressing. In embodiments, the devices comprise a single active agent, and in other embodiments, more than one (e.g., 2, 3, 4, 5, etc.,) active agents can be included in the devices described herein. In suitable embodiments, the active agent (or multiple active agents calculated together) is present in an amount of 0.5% to 2%, 0.5% to 1.9%, 0.5% to 1.8%, 0.5% to 1.7%, 0.5% to 1.6%, 0.5% to 1.5%, 0.5% to 1.4%, 0.5% to 1.3%, 0.5% to 1.2%, 0.5% to 1.1%, 0.5% to 1.0%, 0.5% to 0.9 %, 0.5% to 0.8%, 0.5% to 0.7%, 0.5% to 0.6%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.5% to 10%, 2.5% to 9%, 2.5% to 8%, 2.5% to 7%, 2.5% to 6%, 2.5% to 5%, 2.5% to 4%, 2.5% to 3%, 3% to 10%, 3% to 9%, 3% to 8%, 3% to 7%, 3% to 6%, 3% to 5% or 3% to 4%.

In exemplary embodiments, the active agent is salicylic acid, suitably present at an amount of 0.5% to 2%. More suitably, the salicylic acid is present at an amount of 0.5% to 1.5%, or 0.5%, 0.6%, 0.7%, 0.8%, 0.9%, 1.0%, 1.1%, 1.2%, 1.3%, 1.4% or 1.5%.

In further embodiments, the active agent is benzoyl peroxide, suitably present at an amount of 2.5% to 10%. More suitably, the benzoyl peroxide is present at an amount of 2.5% to 9%, 2.5% to 8%, 2.5% to 7%, 2.5% to 6%, 2.5% to 5%, 2.5% to 4%, 2.5% to 3%.

In still further embodiments, the active agent is sulfur, suitably present at an amount of 3% to 10%. More suitably, the sulfur is present at an amount of 3% to 9%, 3% to 8%, 3% to 7%, 3% to 6%, 3% to 5% or 3% to 4%.

In additional embodiments, the active agent can comprise a combination of Resorcinol 2% in combination with Sulfur or Resorcinol monoacetate In suitable such embodiments, resorcinol is present at an amount of about 1% to about 5%, or about 1% to about 4%, about 1% to about 3%, or about 1%, about 2%, about 3%, about 4% or about 5%. In additional embodiments, the resorcinol monoacetate is present at an amount of about 1% to about 5%, or about 1% to about 4%, about 2% to about 4%, or about 1%, about 2%, about 3%, about 4%, or about 5%. In embodiments where resorcinol or resorcinol monoacetate are utilized, the amount of sulfur is suitably present at about 3% to about 8%, about 3% to about 7%, about 3% to about 6%, about 3% to about 5% or about 3% to about 4%.

Active agents can be associated with the hydrocolloid dressing in various ways known in the art, including for example, dispersed within the hydrocolloid dressing, coated on the hydrocolloid dressing, co-mixed with the components of the hydrocolloid dressing, or otherwise physically contacted with the hydrocolloid dressing so that the dressing and the active agent do not separate from one another until the dressing is applied to skin surface.

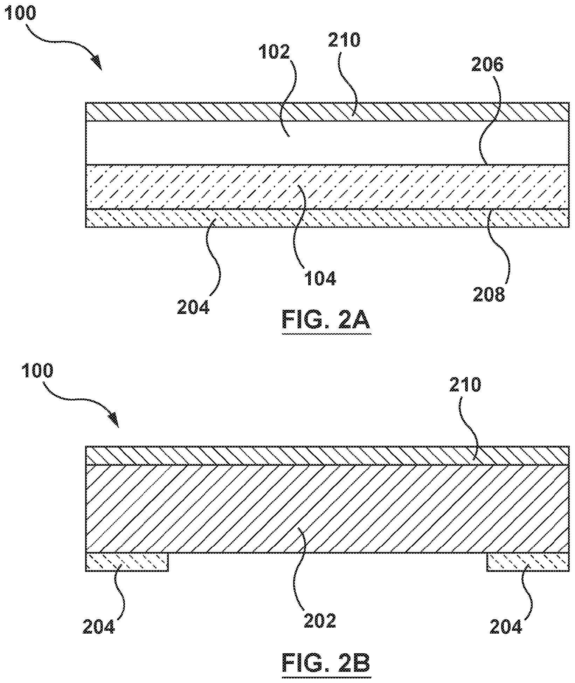

In embodiments, the active agent is associated with an inner surface of the hydrocolloid dressing for contact with a skin surface (106 of FIG. 1). For example, as shown in FIG. 2A, the active agent 104 can be coated on an inner surface 206 of the hydrocolloid dressing 102, or loaded into the hydrocolloid dressing so as to be more concentrated at the inner surface 206. In other embodiments, the active agent is dispersed throughout the hydrocolloid dressing in a uniform or fairly uninform manner (see 202 of FIG. 2B), so as to readily diffuse from the hydrocolloid dressing to the acne blemish. Hydrocolloid dressing 102 also further comprises carrier 210, which suitably is a polyurethane foam or film. Carrier 210 helps provide stability to the hydrocolloid dressing, and also protect the surface of the dressing away from the skin surface (i.e., the non-contact surface) from dirt or other contaminants. The terms "carrier" and "laminate" are used interchangeably herein.

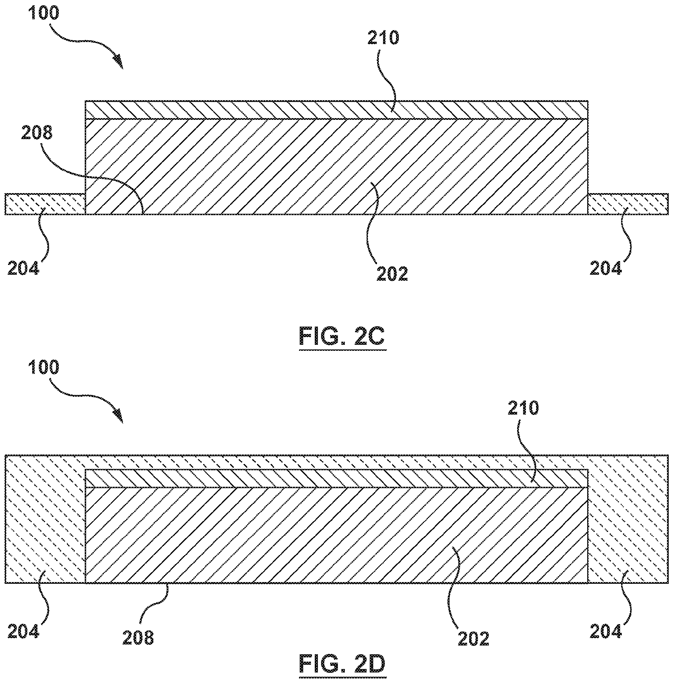

As described herein, the devices comprising the hydrocolloid dressings suitably adhere to the skin surface without any further need for an adhesive or tape. However, in additional embodiments, the inner surface of the devices 208 can further comprise an adhesive 204 for adhering to the skin surface. Adhesive 204 can fully cover the active agent-associated hydrocolloid dressing (FIG. 2A), or can simply be present at the external edges of the hydrocolloid dressing (FIG. 2B), or completely external to the inner surface of the devices 208 (FIG. 2C or FIG. 2D), so as to not interfere with the diffusion of the active agent, and the absorption of oil, pus, etc., from the skin surface.

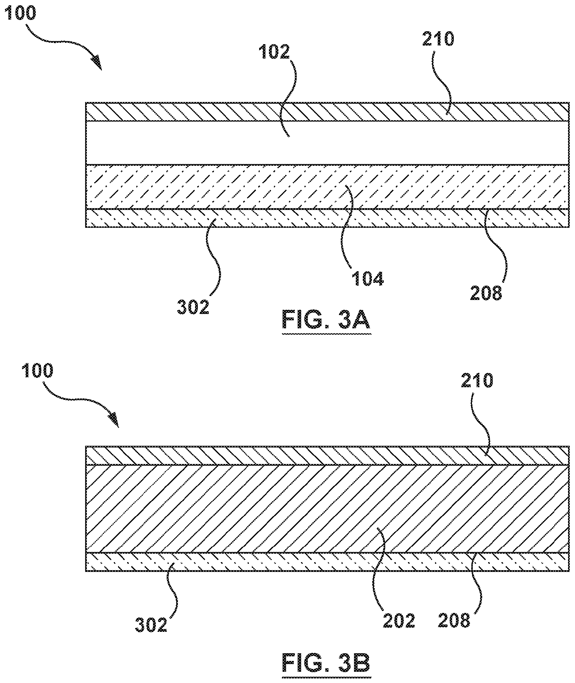

In further embodiments, as shown in FIGs. 3A-3B, the devices described herein 100 further comprises a removable layer 302 covering the inner surface of the devices 208. The terms "removable layer," "removable liner" and "liner" are used interchangeably herein. This removable layer acts as a barrier to dirt and bacteria contacting the device, and also a way to maintain the cleanliness, including sterility, of the devices prior to use on a skin surface. The layer also acts to prevent the devices 100 from sticking to a skin surface prior to a desired use. Removable layer 302 can be a thin layer of paper, cloth, polymer (e.g., polyurethane laminate), plastic, etc., that can readily be removed from the device prior to use. In addition, in embodiments where a further adhesive 204 is used in the devices, removable layer 302 suitably covers the adhesive 204 so as to prevent the device from sticking to a skin surface prior to a desired use, and also maintains the cleanliness of the adhesive.

Also provided herein are devices for treatment of acne, consisting essentially of a hydrocolloid dressing and salicylic acid present at an amount of 0.5% to 1.2%. In devices that consist essentially of the recited components, such devices contain the recited components and those that do not materially affect the basic and novel characteristics of the claimed devices. Components that do not materially affect the basic and novel characteristics of the claimed devices are those that do not limit to ability of the devices to deliver the active agent to the skin surface, while also not reducing the absorption capability of the hydrocolloid dressing. Examples of components that would not materially affect the basic and novel characteristics of the claimed devices, and thus could be included in devices that consist essentially of the recited components, include various botanicals (e.g., aloe vera), scented oils, vitamins (e.g. vitamin A), etc.

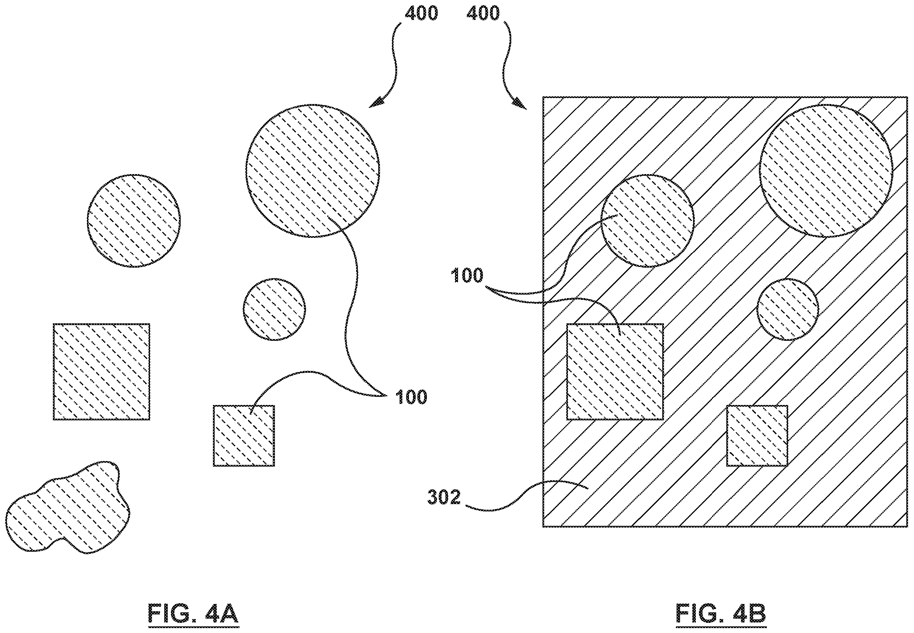

Also provided are systems for treatment of acne. As shown in FIG. 4A, systems 400 suitably comprise a plurality of devices 100. As described herein, suitably each device 100 comprises a removable layer on an inner surface of the device; and a flexible sheet or wafer consisting of i) a hydrocolloid dressing comprising a polyurethane foam or film, an active agent selected from the group consisting of salicylic acid, benzoyl peroxide and sulfur associated with the hydrocolloid dressing, wherein the active agent is present at an amount so as to not reduce the absorption capability of the hydrocolloid dressing, and a removable layer 302 covering an inner surface of device. Suitably, systems 300 include devices where each device is in the form of an application pad having a size of 0.5 cm to 4 cm.

As used herein, plurality refers to more than one of a element, for example, a "plurality" of devices means more than 2, more than 3, 4, 5, 6, 7, 8, 9, 10, etc. Suitably the systems 400 comprise about 5 to about 100 devices 100, for example about 5 to about 50, about 5 to about 40, about 5 to about 30, about 5 to about 20, about 10, about 20, about 30, about 40, or about 50 devices 100.

As used herein "application pad" refers to the ability to separately take each device 100 and apply it to a unique and specific portion of a patient's skin. The size of devices 100 for use in systems 400 suitably ranges from about 0.1 cm to about 10 cm, more suitably from about 0.5 cm to about 10 cm, about 0.5 cm to about 9 cm, about 0.5 cm to about 8 cm, about 0.5 cm to about 7 cm, about 0.5 cm to about 6 cm, about 0.5 cm to about 5 cm, about 0.5 cm to about 4 cm, about 0.5 cm to about 3 cm, about 0.5 cm to about 2 cm, about 0.5 cm to about 2 cm, about 1 cm to about 3 cm, about 0.5 cm, about 1 cm, about 1.5 cm, about 2 cm, about 2.5 cm, about 3 cm, about 3.5 cm, about 4 cm, about 4.5 cm or about 5 cm. Suitably devices of this size are useful on the face or other areas where specific acne blemishes are to be treated.

In further embodiments, the size of devices can be from about 5 cm to about 40 cm, or about 5 cm to about 20 cm, for use on larger areas, for example, the shoulders, back or chest, where it is desirable to cover a larger area, rather than specific individual acne blemishes.

The sizes of devices are suitably measured as the diameter of a circular device, or as the largest dimension of a square, rectangular, triangular, or other regular-shaped device. For irregular-shaped devices, the size is a characteristic dimension that is generally over the largest cross-sectional area of the device. In additional embodiments, the size of the devices can be expressed as a surface area (i.e., the surface area of the inner surface that will be applied to a skin surface), which is suitably in the range of about 0.25 cm² to about 100 cm², more suitably about 0.25 cm² to about 25 cm², or about 1.25 cm² to about 4 cm² for smaller devices, and on the order of about 25 cm² to about 1600 cm², for larger devices.

In embodiments, for example as shown in FIG. 4A, systems 400 suitably comprise a plurality of devices that are separate. FIG. 4A shows a "top view" of the devices, simply illustrating the devices as individual components. In embodiments, each device 100 suitably comprises removable layer 302 covering the inner surface of the device (not shown). In embodiments, such systems can be provided as loose, individual devices, for example in a packaging or box, from which an individual device can be removed. Prior to use then, the removable layer 302 is removed from the device, exposing the hydrocolloid dressing, which can then be applied to a skin surface. If a separate adhesive 204 is used, removal of removable layer 302 also exposes adhesive layer 204 so as to allow it to be applied to a skin surface.

In additional embodiments, as shown for example in FIG. 4B, the systems 400 can comprise at least two of devices 100, which are attached to the same removable layer 302. In such embodiments, removable layer 302 acts as a sheet from which devices 100 can be removed prior to use on a skin surface. Systems 400 such as shown in FIG. 4B can be packaged as multiple sheets, for example in box or other suitable container, prior to removal and use.

In embodiments of systems 400 described herein, devices 100 can have any suitable or desired shape, including for example, square, circle, oval, rectangle, triangle, as well as irregular shapes, and also shapes that have specific configurations for use on different parts of the body, e.g., bridge of the nose, shoulders or back, chest, cheekbones, forehead, chin, etc.

Preparing systems 400 of a plurality of devices 100 is suitably carried out by cutting larger sheets of devices into individual devices. For example, die cutting or kiss cutting can be used to prepare systems 400 of devices 100.

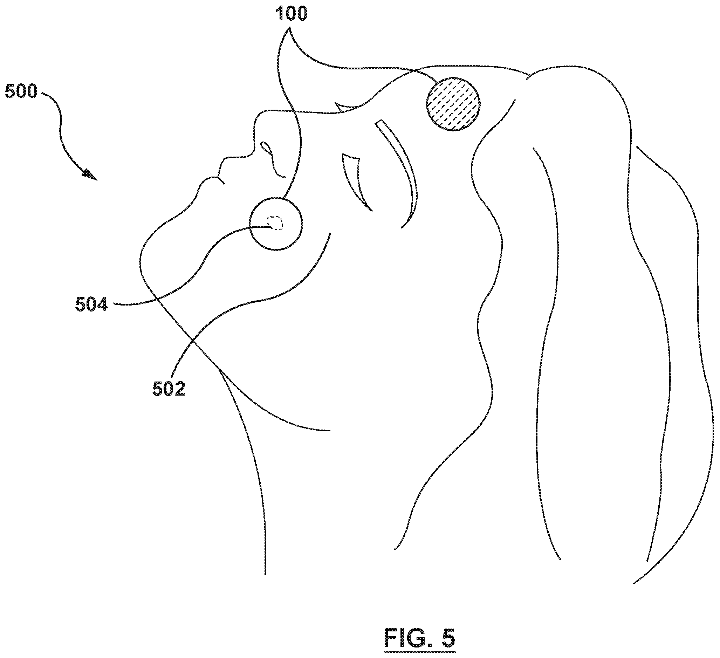

In a further embodiment according to the disclosure, provided herein are methods of treating acne 500, *see* FIG. 5. In suitable embodiments, the methods comprise providing a device 100 as described herein, suitably comprising a hydrocolloid dressing and an active agent selected from the group consisting of salicylic acid, benzoyl peroxide and sulfur associated with the hydrocolloid dressing. As described throughout, the active agent is present at an amount so as to not reduce the absorption capability of the hydrocolloid dressing.

The methods of treating acne further comprise applying the device to a skin surface 502, *see* FIG. 5, the device at least partially covering an acne blemish, e.g., 504 in FIG. 5 (blemish 504 is shown as hidden under device 100). The methods further comprise maintaining the device on the skin surface for a period of at least 1 hour, thereby treating the acne.

Suitably, the skin surface is cleaned (e.g., using soap and/or water, or a suitable cleansing solution or agent) prior to applying the device.

As used herein "acne" refers to occluded pilosebaceous units which develop into lesions including papules, pustules, comedones, and/or cysts on an inflammatory base. Factors involved in acne pathogenesis include: androgens, follicular obstruction, and *Propionobacterium acnes* infection. An "acne blemish" as used herein refers to blackheads (comedones), a whitehead (papule or pustule) as well as cysts.

The period that a device is maintained on a skin surface can be as little as 10-15 minutes, but is suitably at least 1 hour, including maintaining the device on a skin surface for at least 6 hours (e.g., overnight) up to an including 12-24 hours.

Suitable skin surfaces onto which the devices can be applied include any surface where an acne blemish occurs, including for example, the face, neck, back, shoulders, chest, buttocks, trunk, legs, etc. Suitably, the devices and methods described herein are useful in the treatment of acne in mammals, and specifically humans.

The methods of treatment described herein, the treatment is suitably repeated on a daily basis, though can be repeated on semi-daily (i.e., every 2, 3, 4, 5 days) etc., as desired. Suitably the treatment is repeated daily for at least 3 days, or until the acne blemish is no longer noticeable to a user. The methods can be repeated for at least 3-30 days, e.g., about 7-14 days, or about 7-10 days.

In embodiments in which a removable layer 302 is included on an inner surface of the device, the methods further comprise removing the removable layer prior to applying the device 100 to a skin surface 502.

In embodiments, such as shown in FIG. 4A, removable layer 302 is removed from an individual device 100, prior to applying to skin surface 502. In embodiments, such as shown in FIG. 4B, removable layer 302 is removed from an individual device 100, leaving behind the remaining devices 100 on removable layer 302, suitably further comprising additional devices 100 still attached to removable layer 302.

As described herein, applying the device 100 to a skin surface to at least partially cover (suitably completely cover) an acne blemish, allows the hydrocolloid dressing to absorb impurities, including oil, pus, dirt, bacteria, etc., and also allow the active agent (e.g., salicylic acid, benzoyl peroxide, sulfur) to absorb onto the surface of the acne blemish, begin to penetrate the acne blemish, and have a treatment effect on the blemish.

While in suitable embodiments a device can be removed and replaced immediately, in other embodiments, a period of time between removal and replacement is allowed to pass, suitably on the order of 15 minutes to several hours to days, suitably on the order of about 4-18 hours. For example, in embodiments, devices 100 are removed from a skin surface during the day e.g., after waking in the morning, and then reapplied at night, e.g., at bedtime.

The devices and methods described herein, while suitably useful for treatment of acne and acne blemishes, can be useful for application to pores and for treatment of wrinkles, as well as other skin conditions, for example, for use in skin lightening or dark spot removal.

In further embodiments, provided herein are methods of preparing a device as described, the methods comprising providing a solution of an active agent selected from the group consisting of salicylic acid, benzoyl peroxide and sulfur, mixing the solution of the active agent with a paste comprising a hydrocolloid, the mixing conducted at 20-30°C, to form a mixture, and applying the mixture to a removable layer to generate the device. As described herein, it has been determined that the amount of active agent must be selected so as to be present at an amount as to not reduce the absorption capability of the hydrocolloid dressing.

Suitable active agents and components of the devices are described herein.

In further embodiments, the methods further comprise cutting the device into a plurality of separate devices, so as to aid in packaging and use by a patient.

Also provided herein are devices for treatment of acne prepared by the processes described herein.

In embodiments, the methods described herein are used to prepare a device comprising salicylic acid.

The components of the hydrocolloid composition are suitably provided as a powder. The power is combined with water to prepare a paste comprising the hydrocolloid composition.

The paste is then combined with a solution of active agent having the desired final amount active agent (e.g., 0.5% to 1.5% salicylic acid) to create a mixture.

The resulting mixture is mixed using a stiff spatula "swipe" the salicylic acid mass against the sides of the mixing container to avoid entrapment of air in the mix.

As salicylic acid reacts or gels chemically, temperature is an important factor in the setting time. The colder the temperature of the water the longer it takes to set; conversely, the higher the water temperature the faster it sets. Salicylic Acid melting point is about at 158.6°C, with suitable water temperature for solubility at room temperature, or 20-30°C.

Suitably, a hydrocolloid composition (paste) and salicylic acid are mixed in a Banbury mixer at 30 RPM for about 30 minutes. The resulting salicylic acid-containing hydrocolloid adhesive is then converted into a thickness of between about 0.007 inches up to about 0.030 inches, more suitably about 0.010 inches up to about 0.018 inches, or about 0.008 inches up to about 0.025 inches, suitably about 0.015 includes, about 0.016 inches, about 0.017 inches, about 0.018 inches, about 0.019 inches, or about 0.020 inches, including values and ranges in between these ranges.

A hydrocolloid dressing sheet can be cut using a pressure point plaster die-cutting and packaging machine in order produce the hydrocolloid dressing into suitable sizes and shapes. For example, suitable sizes are about 0.5 cm to about 2 cm, or about 1 cm to 1.5 cm, or about 1.1 cm. In exemplary embodiments, each hydrocolloid dressing sheet can produce about 12 devices. These devices can be packaged into various containers or pouches prior to use or for storage.

The thickness of the different dressings is suitably determined using a Wallace thickness gauge, Model S.4 with 20.36 gram additive weight. In order to avoid localized compression of the dressing by the foot of the measuring gauge, a metal disc 20 mm in diameter of known thickness is placed upon the surface of the dressing and the combined thickness of the disc and dressing measured.

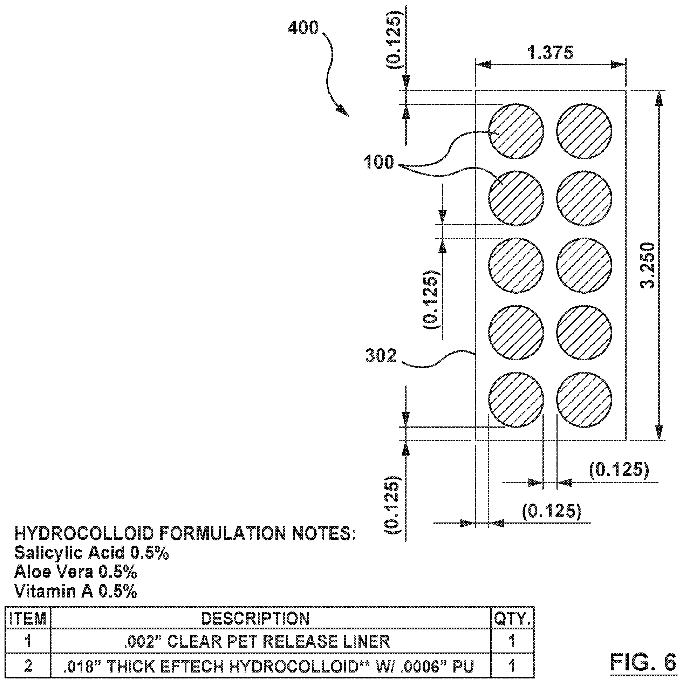

FIG. 6 shows a further system 400 for treatment of acne as described herein. System 400 of FIG. 6 includes a plurality of devices 100 placed on removal layer 302. The system of FIG. 6 includes a hydrocolloid (0.018 inch thick hydrocolloid) and a 0.002 inch clear poly(ethylene terephthalate) (PET) removable layer 302. The device includes 0.5% salicylic acid, 0.5% aloe vera and 0.5% vitamin A. The sizes, dimensions (shown in inches) and orientations of the device are for illustrative purposes only.

### EXAMPLE 1

### Exemplary Construction of Device as Described Herein

The following provides an exemplary construction of a device as described herein.

| **Construction** | | **US Mil¹** | **mm** |
|---|---|---|---|
| **Dressing:** | Hydrocolloid | 20.0 | 0.51 |
| **Laminate (carrier):** | Matte Finish Polyurethane Film | 1.2 | 0.03 |
| **Liner (removable layer):** | 60# White Polycoated Kraft Paper | 4.5 | 0.11 |
| | Total Construction | 25.7 | 0.65 |

| | | | |
|---|---|---|---|
| ¹ A unit of length equal to one thousandth (10⁻³) of an inch (0.0254 millimeter). | | | |

An active agent as described herein is then dissolved in a suitable solvent and mixed with hydrocolloid to generate the devices. Suitable active agents and percentages are described herein.

The exemplary device provides the following features and benefits: medium initial tack aids in placement of product on skin; flexible and conformable; light in color and virtually transparent; no animal derived components; serializable by electron beam (EB) or Gamma radiation, as well as other means.

The devices were determined in compliance with ISO 10993, with regard to cytotoxicity, sensitization and irritation.

The exemplary devices were found to have the following physical characteristics.

| **Adhesive Properties** | |
|---|---|
| **Peel Adhesion** | Test Method: PTSC-101F |
| Product 90° 12 in / min | |
| **Substrate** | **US Oz** / **in** |
| SS | 60-120 |
| **Absorption** | 1" Diameter Sample |
| Product in Saline Solution | |
| **Substrate** | **% by weight** / **24 hours** |
| Product | > 300% |

The devices have been stored without degradation for a period of at least 24 months from date of manufacture at 72°F (21°C), 25% relative humidity, out of direct sunlight, in original packaging.

It will be readily apparent to one of ordinary skill in the relevant arts that other suitable modifications and adaptations to the methods and applications described herein can be made without departing from the scope of any of the embodiments. The following examples are included herewith for purposes of illustration only and are not intended to be limiting.

It is to be understood that while certain embodiments have been illustrated and described herein, the claims are not to be limited to the specific forms or arrangement of parts described and shown. In the specification, there have been disclosed illustrative embodiments and, although specific terms are employed, they are used in a generic and descriptive sense only and not for purposes of limitation. Modifications and variations of the embodiments are possible in light of the above teachings. It is therefore to be understood that the embodiments may be practiced otherwise than as specifically described.

While various embodiments have been described above, it should be understood that they have been presented only as illustrations and examples of the present technology, and not by way of limitation. It will be apparent to persons skilled in the relevant art that various changes in form and detail can be made therein without departing from the spirit and scope of the present technology. Thus, the breadth and scope of the present technology should not be limited by any of the above-described embodiments, but should be defined only in accordance with the appended claims and their equivalents. It will also be understood that each feature of each embodiment discussed herein, and of each reference cited herein, can be used in combination with the features of any other embodiment.

## Claims

1. A device for treatment of acne, comprising:
a removable layer on an inner surface of the device; and
a flexible sheet or wafer consisting of
a. a hydrocolloid dressing comprising a polyurethane foam or film; and
b. an active agent selected from the group consisting of salicylic acid, benzoyl peroxide and sulfur associated with the hydrocolloid dressing,
wherein the active agent is present at an amount so as to not reduce the absorption capability of the hydrocolloid dressing,
wherein benzoyl peroxide is present at an amount of 2.5% to 10%, or the sulfur is present at an amount of 3% to 10%.

2. The device of claim 1, wherein the active agent is salicylic acid present at an amount of 0.5% to 2%, preferably wherein the salicylic acid is present at an amount of 0.5% to 1.5%.

3. The device of any one of claims 1-2, wherein the inner surface of the device further comprises an adhesive for adhering to the skin surface.

4. A device for treatment of acne, consisting essentially of:
a. a hydrocolloid dressing; and
b. salicylic acid present at an amount of 0.5% to 1.5%,
wherein the hydrocolloid dressing comprises a polyurethane foam or film.

5. A system for treatment of acne, comprising:
a. a plurality of devices according to claim 1, each device comprising:
a removable layer on an inner surface of the device; and
a flexible sheet or wafer consisting of
i. a hydrocolloid dressing comprising a polyurethane foam or film;
ii. an active agent selected from the group consisting of salicylic acid, benzoyl peroxide and sulfur associated with the hydrocolloid dressing,
wherein the active agent is present at an amount so as to not reduce the absorption capability of the hydrocolloid dressing; and
wherein the removable layer covers an inner surface of device,
wherein each device is in the form of an application pad having a size of 0.5 cm to 4 cm.

6. The system of claim 5, wherein the plurality of devices are separate, and each comprises the removable layer covering the inner surface of the device, and preferably wherein at least two of the devices are attached to the same removable layer.

7. The system of claim 5 or claim 6, wherein the plurality of devices have square, circle or oval shapes.

8. A method of preparing a device for treatment of acne according to claim 1, comprising:
a. providing a solution of an active agent selected from the group consisting of salicylic acid, benzoyl peroxide and sulfur;
b. mixing the solution of the active agent with a paste comprising a hydrocolloid, the mixing conducted at 20-30°C, to form a mixture; and
c. applying the mixture to a removable layer to generate the device,
wherein the active agent is present at an amount so as to not reduce the absorption capability of the hydrocolloid dressing

9. The method of claim 8, wherein the amount of salicylic acid is 0.5% to 1.5%, preferably wherein the method further comprises cutting the device into a plurality of separate devices.

10. A device for treatment of acne prepared by the process of claim 8.

## Patentansprüche

1. Vorrichtung zur Behandlung von Akne, umfassend:
eine abnehmbare Schicht auf einer inneren Oberfläche der Vorrichtung; und
ein flexibles Blatt oder Wafer, bestehend aus
a. einen Hydrokolloidverband, der einen Polyurethanschaum oder -film umfasst; und
b. einen Wirkstoff, ausgewählt aus der Gruppe bestehend aus Salicylsäure, Benzoylperoxid und Schwefel, der mit dem Hydrokolloidverband verbunden ist,
wobei der Wirkstoff in einer solchen Menge vorhanden ist, dass er die Absorptionsfähigkeit des Hydrokolloid-Verbandes nicht verringert,
wobei Benzoylperoxid in einer Menge von 2,5% bis 10% oder der Schwefel in einer Menge von 3% bis 10% vorhanden ist.

2. Vorrichtung nach Anspruch 1, wobei der Wirkstoff Salicylsäure ist, die in einer Menge von 0,5 % bis 2 % vorhanden ist, vorzugsweise wobei die Salicylsäure in einer Menge von 0,5 % bis 1,5 % vorhanden ist.

3. Die Vorrichtung nach einem der Ansprüche 1 bis 3, wobei die innere Oberfläche der Vorrichtung außerdem einen Klebstoff zum Anhaften an der Hautoberfläche umfasst.

4. Vorrichtung zur Behandlung von Akne, im wesentlichen bestehend aus:
a. einen Hydrokolloid-Verband; und
b. Salicylsäure in einer Menge von 0,5% bis 1,5%,
wobei der Hydrokolloid-Verband einen Polyurethan-Schaum oder -Film umfasst.

5. System zur Behandlung von Akne, umfassend:
a. eine Vielzahl von Vorrichtungen nach Anspruch 1, wobei jede Vorrichtung umfasst:
eine abnehmbare Schicht auf einer inneren Oberfläche der Vorrichtung; und
ein flexibles Blatt oder Wafer, bestehend aus
i. einen Hydrokolloid-Verband, der einen Polyurethanschaum oder -film enthält;
ii. einen Wirkstoff, ausgewählt aus der Gruppe bestehend aus Salicylsäure, Benzoylperoxid und Schwefel, der mit dem Hydrokolloidverband verbunden ist,
wobei der Wirkstoff in einer solchen Menge vorhanden ist, dass er die Absorptionsfähigkeit des Hydrokolloid-Verbandes nicht verringert; und
wobei die abnehmbare Schicht eine innere Oberfläche der Vorrichtung bedeckt,
wobei jede Vorrichtung die Form eines Applikationspads mit einer Größe von 0,5 cm bis 4 cm hat.

6. System nach Anspruch 5, wobei die mehreren Vorrichtungen getrennt sind und jede die abnehmbare Schicht umfasst, die die innere Oberfläche der Vorrichtung bedeckt, und wobei vorzugsweise mindestens zwei der Vorrichtungen an der gleichen abnehmbaren Schicht befestigt sind.

7. Das System nach Anspruch 5 oder Anspruch 6, wobei die Mehrzahl der Geräte eine quadratische, kreisförmige oder ovale Form hat.

8. Verfahren zur Herstellung einer Vorrichtung zur Behandlung von Akne nach Anspruch 1, umfassend:
a. Bereitstellung einer Lösung eines Wirkstoffs aus der Gruppe bestehend aus Salicylsäure, Benzoylperoxid und Schwefel;
b. Mischen der Lösung des Wirkstoffs mit einer Paste, die ein Hydrokolloid enthält, wobei das Mischen bei 20-30 °C erfolgt, um eine Mischung zu bilden; und
c. Auftragen der Mischung auf eine abnehmbare Schicht, um die Vorrichtung zu erzeugen,
wobei der Wirkstoff in einer solchen Menge vorhanden ist, dass er die Absorptionsfähigkeit des Hydrokolloid-Verbandes nicht verringert

9. Verfahren nach Anspruch 8, wobei die Menge an Salicylsäure 0,5 % bis 1,5 % beträgt, wobei das Verfahren vorzugsweise weiterhin das Zerschneiden der Vorrichtung in eine Vielzahl von separaten Vorrichtungen umfasst.

10. Vorrichtung zur Behandlung von Akne, hergestellt nach dem Verfahren von Anspruch 8.

## Revendications

1. Dispositif pour le traitement de l'acné, comprenant :
une couche amovible sur une surface intérieure du dispositif ; et
une feuille ou une plaquette flexible constituée de
a. un pansement hydrocolloïde comprenant une mousse ou un film de polyuréthane ; et
b. un agent actif choisi dans le groupe constitué de l'acide salicylique, du peroxyde de benzoyle et du soufre associé au pansement hydrocolloïde ,
dans lequel l'agent actif est présent en quantité telle qu'il ne réduit pas la capacité d'absorption du pansement hydrocolloïde,
dans lequel peroxyde de benzoyle est présent dans une proportion de 2,5 % à 10 %, ou le soufre est présent dans une proportion de 3 % à 10 %.

2. Le dispositif de la revendication 1, dans lequel l'agent actif est l'acide salicylique présent en quantité de 0,5 % à 2 %, de préférence dans lequel l'acide salicylique est présent en quantité de 0,5 % à 1,5 %.

3. Le dispositif de l'une des revendications 1-2, dans lequel la surface intérieure du dispositif comprend en outre un adhésif destiné à adhérer à la surface de la peau.

4. Dispositif pour le traitement de l'acné, consistant essentiellement en.. :
a. un pansement hydrocolloïde ; et
b. acide salicylique présent à raison de 0,5 % à 1,5 %,
dans lequel le pansement hydrocolloïde comprend une mousse ou un film de polyuréthane.

5. Système de traitement de l'acné comprenant
a. une pluralité de dispositifs selon la revendication 1, chaque dispositif comprenant :
une couche amovible sur une surface intérieure du dispositif ; et
une feuille ou une plaquette flexible constituée de
i. un pansement hydrocolloïde comprenant une mousse ou un film de polyuréthane ;
ii. un agent actif choisi dans le groupe constitué de l'acide salicylique, du peroxyde de benzoyle et du soufre, associé au pansement hydrocolloïde,
dans lequel l'agent actif est présent en quantité telle qu'il ne réduit pas la capacité d'absorption du pansement hydrocolloïde ; et
dans lequel la couche amovible recouvre une surface intérieure de l'appareil,
dans lequel chaque dispositif se présente sous la forme d'un tampon d'application d'une taille comprise entre 0,5 cm et 4 cm.

6. Le système de la revendication 5, dans lequel la pluralité de dispositifs sont séparés, et chacun comprend la couche amovible couvrant la surface intérieure du dispositif, et de préférence dans lequel au moins deux des dispositifs sont attachés à la même couche amovible.

7. Le système de la revendication 5 ou de la revendication 6, dans lequel la pluralité de dispositifs a une forme carrée, circulaire ou ovale.

8. Méthode de préparation d'un dispositif de traitement de l'acné selon la revendication 1, comprenant:
a. fournir une solution d'un agent actif choisi dans le groupe constitué de l'acide salicylique, du peroxyde de benzoyle et du soufre ;
b. mélanger la solution d'agent actif avec une pâte comprenant un hydrocolloïde, le mélange étant effectué à 20-30 °C, pour former un mélange ; et
c. appliquer le mélange sur une couche amovible pour produire le dispositif, dans lequel l'agent actif est présent en quantité telle qu'il ne réduit pas la capacité d'absorption du pansement hydrocolloïde

9. La méthode de la revendication 8, dans laquelle la quantité d'acide salicylique est de 0,5 % à 1,5 %, de préférence dans laquelle la méthode comprend en outre le découpage du dispositif en plusieurs dispositifs séparés.

10. Dispositif pour le traitement de l'acné préparé selon le procédé de la revendication 8.
